# EUROPEAN PATENT SPECIFICATION

(11) **EP 1 032 386 B1**
(45) Date of publication and mention of the grant of the patent: **26.02.2003**
(21) Application number: 98960157.0
(22) Date of filing: 10.11.1998
(51) Int. Cl.: A61K 31/42, A61P 19/02

(54) **USE OF OXAZOLIDINONE DERIVATIVES FOR TREATING ARTHRITIS**
VERWENDUNG VON OXAZOLIDINONDERIVATE ZUR BEHANDLUNG VON ARTHRITIS
UTILISATION DE DERIVES D'OXAZOLIDINONE POUR TRAITER L'ARTHRITE

(30) Priority: 18.11.1997 US 65689 P; 16.01.1998 US 71297 P; 04.02.1998 US 73662 P; 19.02.1998 US 75247 P; 12.03.1998 US 77672 P
(43) Date of publication of application: 06.09.2000
(60) Divisional application: 02028171.3
(73) Proprietor: PHARMACIA & UPJOHN COMPANY, Kalamazoo, Michigan 49001 (US)
(72) Inventor: BATTS, Donald, H., Kalamazoo, MI 49008 (US); ULRICH, Roger G., Waukegan, IL 60085 (US)
(74) Representative: Perry, Robert Edward
(86) International application number: US9823233
(87) International publication number: WO99025344

(56) References cited:
- WO-A-93/09103
- WO-A-93/23384
- WO-A-95/14684
- US-A- 5 688 792

## Description

### 1. Field of the Invention

The present invention is the use of known oxazolidinones for the manufacture of a medicament for the treatment of arthritis.

### 2. Description of the Related Art

US Patents 5,164,510, 5,231,188, 5,565,571, 5,652,238 and 5,688,792 all disclose various oxazolidinone antibiotics which are well known to those skilled in the art.

Arthritis, inflammation of the joint tissues, has numerous causes including bacterial infection (septic arthritis), degeneration of articular surfaces (osteoarthritis), immunologic reaction against joint tissues (rheumatoid arthritis), crystal induced arthritis (gouty arthritis, pseudogout) and other miscellaneous causes (Reiter's syndrome, etc). One common thread in all of these is inflammation in and around the joint. Present day therapeutics for arthritis are not curative unless the arthritis is infectious and the underlying pathogen is eliminated by an antibiotic. For other types of arthritis medications can reduce pain or inflammation but do not cure the disease. The OXAZOLIDINONEs can be used to treat the inflammation and pain caused by arthritis, including those types of arthritis which are not caused by infection. This is important since all of the medications presently available to treat arthritis have severe side effects. Steroidal medications (glucocorticoids like prednisone and cortisol) give stomach ulcers, cataracts, reduced resistance to infection, weight gain and thinning of skin. Non-steroidal anti-inflammatory drugs (NSAIDS such as indomethacin or ibuprofen among others) can cause stomach ulcers, reduced kidney function and bone marrow effects. Chemotherapeutic agents (like methotrexate) can have severe adverse effects on bone marrow and liver function. The OXAZOLIDINONEs provide the opportunity for relief of symptoms of inflammation for patients intolerant of other types of arthritis medications.

### SUMMARY OF INVENTION

Disclosed is the use of an effective amount of an oxazolidinone selected from the group consisting of:
(S)-N-[[3-[3-fluoro-4-[4-(hydroxyacetyl)-1-piperazinyl]-phenyl]-2-oxo-5-oxazolidinyl]methyl]acetamide,
(S)-N-[[3-[3-fluoro-4-(4-morpholinyl)phenyl]-2-oxo-5-oxazolidinyl]methyl]acetamide,
[4(S)-cis]-(-)-N-[[3-[3-Fluoro-4-(tetrahydro-1-oxido-2H-thiopyran-4-yl)phenyl]-2-oxo-5-oxazolidinyl]methyl]acetamide,
N-((5S)-3-(3-fluoro-4-(4-(2-fluoroethyl)-3-oxopiperazin-1-yl)phenyl)-2-oxooxazolidin-5-ylmethyl)acetamide,
(S)-N-[[3-[5-(3-pyridyl)thiophen-2-yl]-2-oxo-5-oxazolidinyl]methyl]acetamide and
(S)-N-[[3-[5-(4-pyridyl)pyrid-2-yl]-2-oxo-5-oxazolidinyl]methyl]acetamide and pharmaceutically acceptable salts thereof for the manufacture of a medicament for the treatment or arthritis.

### DETAILED DESCRIPTION OF THE INVENTION

The oxazolidinones of the present invention are known, see EXAMPLES 1 thru 6 (OXAZOLIDINONEs) and pharmaceutically acceptable salts thereof.

Suitable pharmaceutical acceptable salts include the acid addition salts from the both inorganic and organic acids including hydrochloric, hydrobromic, sulfuric, phosphoric, sulfonic acids, methanesulfonic, gluconic, galacturonic, citratic, oxylatic and acetic.

The person being treated for arthritis must not have a gram positive bacterial infection at the time of being treated.

In treating arthritis, the OXAZOLIDINONEs can either be used individually or in combination with each other or in combination with non-OXAZOLIDINONEs.

In treating arthritis, the OXAZOLIDINONEs are administered orally, parenterally (IV), topically or rectally.

When administered orally, the OXAZOLIDINONEs can be administered in tablet, capsule or liquid (suspension, syrup or solution) dosage form. Regardless of the dosage form, an anti-arthritis effective amount of the OXAZOLIDINONEs is from about 50 mg to about 1,200 mg/day. It is preferred that the OXAZOLIDINONEs be given in two or more divided doses, more preferably in two divided doses.

When administered parenterally, the OXAZOLIDINONEs should be administered IV. The IV infusion should be adjusted such that the flow rate delivers an anti-arthritis effective amount of from about 50 mg to about 1,200 mg/day. This can be done by a continuous infusion, or as divided doses given as short term infusions of 30-90 minutes, preferably twice a day.

When applied topically, the OXAZOLIDINONEs can be applied in many different pharmaceutical dosage forms all well known to those skilled in the art. These include a solution, cream, ointment, gel, lotion, suspension or emulsion. Regardless of the pharmaceutical dosage form selected, the concentration of the OXAZOLIDINONEs should be from about 0.01% to about 10% (wt/wt). Regardless of the pharmaceutical dosage form, the topical formulation should be applied one to four times daily.

When administered rectally, the OXAZOLIDINONEs should be administered as a suppository which delivers an anti-arthritis effective amount of from about 50 mg to about 1,200 mg/day.

When administered systemically, whether orally, parenterally or rectally the OXAZOLIDINONEs should be administered either continuously or in cyclic courses of 7-28 days every 1 to 12 months. When administered topically, the OXAZOLIDINONEs should be applied either daily for 7 to 28 days every 1 - 12 months.

The exact dosage and frequency of administration depends on the particular OXAZOLIDINONE used, the severity of the condition being treated, the age, weight, general physical condition of the particular patient, other medication the individual may be taking as is well known to those skilled in the art and can be more accurately determined by measuring the blood level or concentration of the OXAZOLIDINONE in the patient's blood and/or the patient's response to the particular condition being treated.

### DEFINITIONS AND CONVENTIONS

The definitions and explanations below are for the terms as used throughout this entire document including both the specification and the claims.

### DEFINITIONS

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

When the solubility of a solid in a solvent is used the ratio of the solid to the solvent is weight/volume (wt/v).

When the % active ingredient of a pharmaceutical formulation is set forth, it is the ratio of the active ingredient of the entire pharmaceutical formulation and is expressed as weight/weight (wt/wt).

Alcohol refers to ethyl alcohol.

IV refers to parenteral administration by the intravenous route.

OXAZOLIDINONES refers to the compounds of EXAMPLES 1 thru 6.

Anti-cytotoxic refers to reducing and/or preventing normal cell death.

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### EXAMPLE 1 (S)-N-[[3-[3-Fluoro-4-[4-(hydroxyacetyl)-1-piperazinyl]-phenyl]-2-oxo-5-oxazolidinyl]methyl]acetamide

(S)-N-[[3-[3-Fluoro-4-[4-(hydroxyacetyl)-1-piperazinyl]-phenyl]-2-oxo-5-oxazolidinyl]methyl]acetamide is known, see US Patent 5,652,238, EXAMPLE 1.

### EXAMPLE 2 (S)-N-[[3-[3-Fluoro-4-(4-morpholinyl)phenyl]-2-oxo-5-oxazolidinyl]methyl]acetamide

(S)-N-[[3-[3-Fluoro-4-(4-morpholinyl)phenyl]-2-oxo-5-oxazolidinyl]methyl]acetamide is known, see US Patent 5,688,792, EXAMPLE 5.

### EXAMPLE 3 [4(S)-cis]-(-)-N-[[3-[3-Fluoro-4-(tetrahydro-1-oxido-2H-thiopyran-4-yl)phenyl]-2-oxo-5-oxazolidinyl]methyl]acetamide

A mixture of (S)-(-)-N-[[3-(3-fluoro-4-(3,6-dihydro-2H-thiopyran-4-yl)phenyl]-2-oxo-5-oxazolidinyl]methyl]acetamide S-oxide (International Publication No. WO 97/09328, 4.50 g) and platinum oxide (697 mg) in methanol (164 ml) is shaken on the Parr apparatus under a hydrogen atmosphere at 40 psi for 18 hours. The catalyst is then removed by filtration through Celite, and the filtrate is concentrated under reduced pressure and the residue chromatographed on silica gel (230 - 400 mesh, 350 g), eluting with a gradient of methanol/methylene chloride (3/97 - 7/93). The appropriate fractions (those fractions with an R_{f} = 0.44 by TLC; methanol/chloroform, 10/90) are pooled and concentrated to give the title compound, mp 203 - 204°.

### EXAMPLE 4 N-((5S)-3-(3-Fluoro-4-(4-(2-fluoroethyl)-3-oxopiperazin-1-yl)phenyl)-2-oxooxazolidin-5-ylmethyl)acetamide

N-((5S)-3-(3-Fluoro-4-(4-(2-fluoroethyl)-3-oxopiperazin-1-yl)phenyl)-2-oxooxazolidin-5-ylmethyl)acetamide is known, see International Publication WO97/27188 (Example 4).

1-*t*-Butoxycarbonyl-3-oxopiperazine (21.6 g) is dissolved in dry DMF (500 ml) and potassium *t*-butoxide (24.2 g) is added. The mixture is stirred at 20-25° for 30 minutes, then 1-(4-methylphenylsulfonyloxy)-2-fluoroethane (*J. Med. Chem., 23(9),* 985-90 (1980), 25.9 g) is added and stirring continued at the same temperature for 24 hours. The solvent is removed and the residue partitioned between ethyl acetate and water. The organic phase is washed with water and concentrated. The residue is dissolved in isopropanol and diluted with iso-hexane forming a precipitate which is removed by filtration. The mixture is chromatographed (silica; eluting with a gradient increasing in polarity from 0 to 50% isopropanol in iso-hexane) to give 1-*t*-butoxycarbonyl-4-(2-fluoroethyl)-3-oxopiperazine.

1-*t*-Butoxycarbonyl-4-(2-fluoroethyl)-3-oxopiperazine (6.65 g) is dissolved in dichloromethane (500 ml), cooled in an ice-bath and trifluoroacetic acid (150 ml) added. The mixture is stirred at the same temperature for 2 hours. The solvent is removed to give a crude product which is dissolved in the minimum volume of ethyl acetate. Slow addition of ether causes precipitation of 1-(2-fluoroethyl)-2-oxopiperazine as the mono trifluoroacetic acid salt.

1-(2-Fluoroethyl)-2-oxopiperazine trifluoroacetate (6.1 g) is dissolved in acetonitrile (100 ml). N,N-Diisopropylethylamine (13 ml) is added to the mixture, followed by 3,4-difluoronitrobenzene (3.39 g) and the mixture heated to reflux for 18 hours. The solvent is removed and the residue chromatographed (silica; eluting with a gradient increasing in polarity from 0 to 4% methanol in dichloromethane) to give 3-fluoro-4-(4-{2-fluoroethyl}-3-oxopiperazin-1-yl)nitrobenzene.

3-Fluoro-4-(4-{2-fluoroethyl)-3-oxopiperazin-1-yl)nitrobenzene (4.35 g) is dissolved in a mixture of ethyl acetate (250 ml) and DMF (5 ml), and the solution flushed with argon. Palladium (10% on carbon, 200 mg) is added and the mixture hydrogenated under ambient pressure. After gas uptake had ceased, the mixture is filtered through celite and solvent removed. The residue is taken up in ethyl acetate, washed twice with water, dried over magnesium sulfate and the solvent is removed to give 5-amino-2-[4-(2-fluoroethyl)-3-oxopiperazin-1-yl]fluorobenzene which is used without further purification.

5-Amino-2-(4-[2-fluoroethyl]-3-oxopiperazin-1-yl)fluorobenzene (2.6 g) is dissolved in dry dichloromethane (50 ml) under argon. Pyridine (1.03 ml) is added, and the mixture cooled to -20°. Benzyl chloroformate (1.6 ml) is added and the mixture stirred for 10 minutes at -20°, before allowing the temperature to rise to 20-25° over 1.5 hours. The solvents are removed and the residue is dissolved in dichloromethane and washed with sodium bicarbonate solution. After drying over magnesium sulfate and removal of the solvent, the residue is chromatographed (silica, eluting with a gradient increasing in polarity from 0 to 5% methanol in dichloromethane) to give 6-benzyloxycarbonylamino-2-(4-[2-fluoroethyl]-3-oxopiperazin-1-yl)fluorobenzene.

A solution of lithium *t*-butoxide is prepared by addition of *n*-butyllithium (1.6 M in hexane, 2.9 ml) to a stirred solution of *t*-butanol (0.43 g) in anhydrous THF (10 ml) at -10° under argon. After cooling to -70°, a solution of 5-benzyloxycarbonylamino-2-(4-[2-fluoroethyl]-3-oxopiperazin-1-yl)fluorobenzene (1.5 g) in dry THF (15 ml) is added. After 10 minutes, (R)-glycidylbutyrate (0.67 g) in dry THF (15 ml) is added to the resulting mixture, and stirring continued at -70° for 15 minutes, before allowing the temperature to rise to 20-25° over 16 hours. Methanol (10 ml) is added, followed by saturated sodium bicarbonate solution (20 ml) and water (10 ml). The organic phase is separated and extracted into ethyl acetate (3 x 25 ml), washed with saline and dried over magnesium sulfate. The solvent is removed and the residue purified by chromatography (silica; eluting with a gradient increasing in polarity from 0 to 3% methanol in dichloromethane) to give (5R)-3-(3-fluoro-4-[4-(2-fluoroethyl)-3-oxopiperazin-1-yl]phenyl)-5-hydroxymethyloxazolidin-2-one.

(5R)-3-(3-Fluoro-4-[4-(2-fluoroethyl)-3-oxopiperazin-1-yl)phenyl)-5-hydroxymethyloxazolidin-2-one (0.8 g) is dissolved in pyridine (15 ml) and the mixture cooled to 0°. Triethylamine (0.38 ml) and methanesulfonyl chloride (0.19 ml) are added to the mixture, and stirring continued at 20-25° for 2 hours. The solvent is removed and the residue dissolved in dichloromethane, washed with water, saline, dried over magnesium sulfate and concentrated. The resulting residue is triturated with ether to give (5R)-3-(3-fluoro-4-[4-(2-fluoroethyl)-3-oxopiperazin-1-yl]phenyl)-5-(methanesulfonyloxymethyl)oxazolidin-2-one (0.76 g) which is used without further purification.

(5R)-3-(3-Fluoro-4-[4-(2-fluoroethyl)-3-oxopiperazin-1-yl]-5-(methanesulfonyloxymethyl)oxazolidin-2-one (719 mg) is dissolved in dry DMF (15 ml) and sodium azide (647 mg) is added to the mixture. The mixture is heated at 80° for 6 hrs and then concentrated to dryness. The resulting residue is dissolved in ethyl acetate, washed twice with water, and dried over magnesium sulfate Removal of the solvent gives (5R)-5-azidomethyl-3-(3-fluoro-4-(4-(2-fluoroethyl)-3-oxopiperazin-1-yl)phenyl)oxazolidin-2-one (413 mg) which is used without further purification.

(5R)-5-Azidomethyl-3-(3-fluoro-4-[4-(2-fluoroethyl)-3-oxopiperazin-1-yl]phenyl)oxazolidin-2-one (360 mg) is dissolved in dry DMF (20 ml) and the mixture purged with argon. Palladium (10% on carbon, 72 mg) is added, followed by acetic anhydride (0.17 ml) and the mixture stirred at 20-25° under hydrogen confined in a balloon for 3 hr. The mixture is filtered through celite, concentrated to dryness and partitioned between ethyl acetate and water. The organic extract is washed with saline, dried over magnesium sulfate and concentrated. The residue is chromatographed (silica gel; eluting with a gradient increasing in polarity from 0 to 2.5% methanol/dichloromethane). The appropriate fractions are pooled and concentrated to give the title compound.

### EXAMPLE 5 (S)-N-[[3-[5-(3-Pyridyl)thiophen-2-yl]-2-oxo-5-oxazolidinyl]methyl]acetamide

(S)-N-[[3-[5-(3-Pyridyl)thiophen-2-yl])-2-oxo-5-oxazolidinyl)methyl]acetamide is known, see US Patent 5,698,574 (Example 124).

### EXAMPLE 6 (S)-N-[[3-[5-(4-Pyridyl)pyrid-2-yl]-2-oxo-5-oxazolidinyl]methyl]acetamide hydrochloride

(S)-N-[[3-[5-(4-Pyridyl)pyrid-2-yl]-2-oxo-5-oxazolidinyl]methyl]acetamide hydrochloride is prepared following the general procedure of US Patent 5,627,181 EXAMPLES 36 and 52 and making non-critical variations but using a 4-pyridinyl adduct.

### EXAMPLE A 50 Year Old White Male - Arthritis

A 50 year old white male (Subject No 1912) who had a history of chronic obstructive pulmonary disease (i.e. he was a smoker), hypertension, coronary artery disease and arthritis of the "legs, arms and shoulders" for which he took non steroidal antiinflammatory agents including aspirin, developed community acquired pneumonia with *Streptococcus pneumoniae* as evidenced by an abnormal chest X-ray. He is treated with 625 mg of (S)-N-[[3-[3-fluoro-4-(4-morpholinyl)phenyl]-2-oxo-5-oxazolidinyl]methyl]acetamide twice daily intravenously for 3 days followed by 8 days of oral (S)-N-[[3-[3-fluoro-4-(4-morpholinyl)phenyl]-2-oxo-5-oxazolidinyl]methyl]acetamide at the same dose.

At long term follow up the patient stated that his arthritis was gone and he was taking no medications.

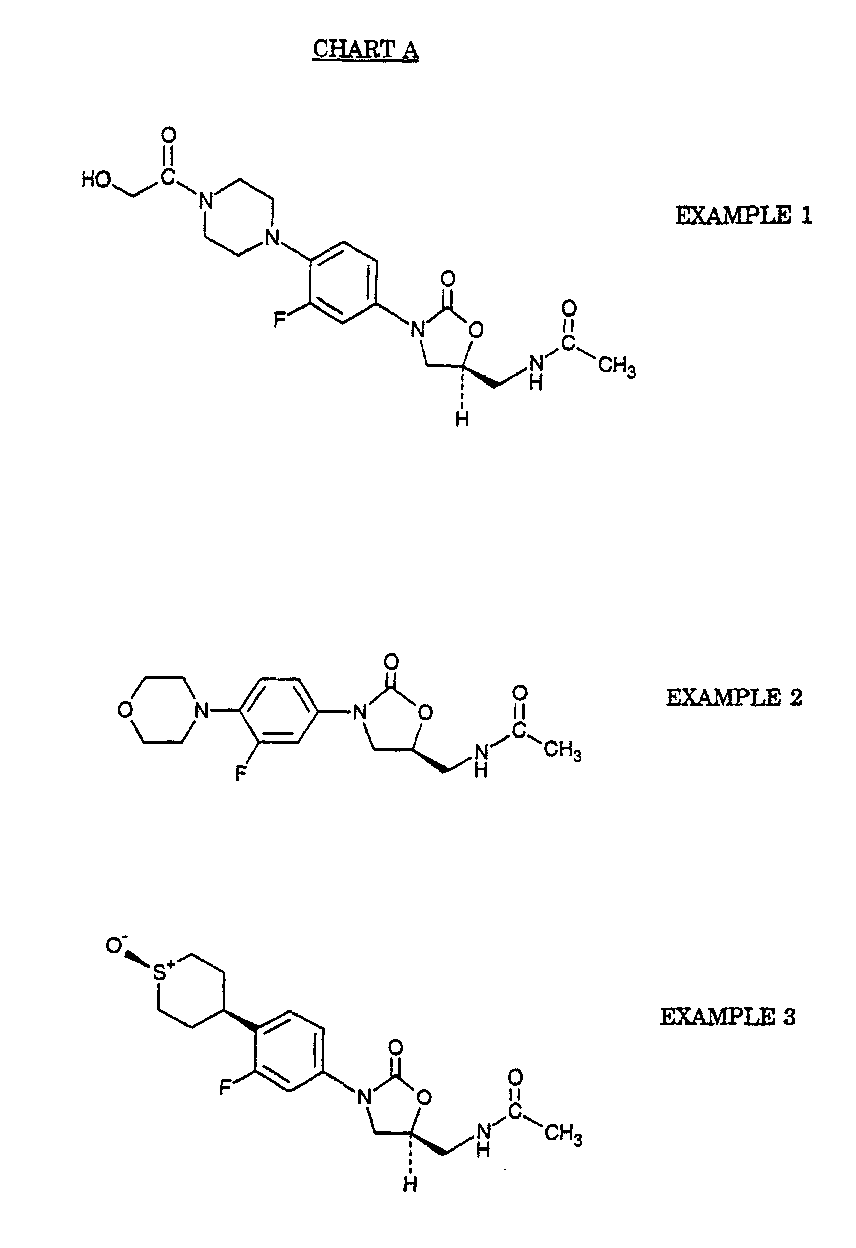

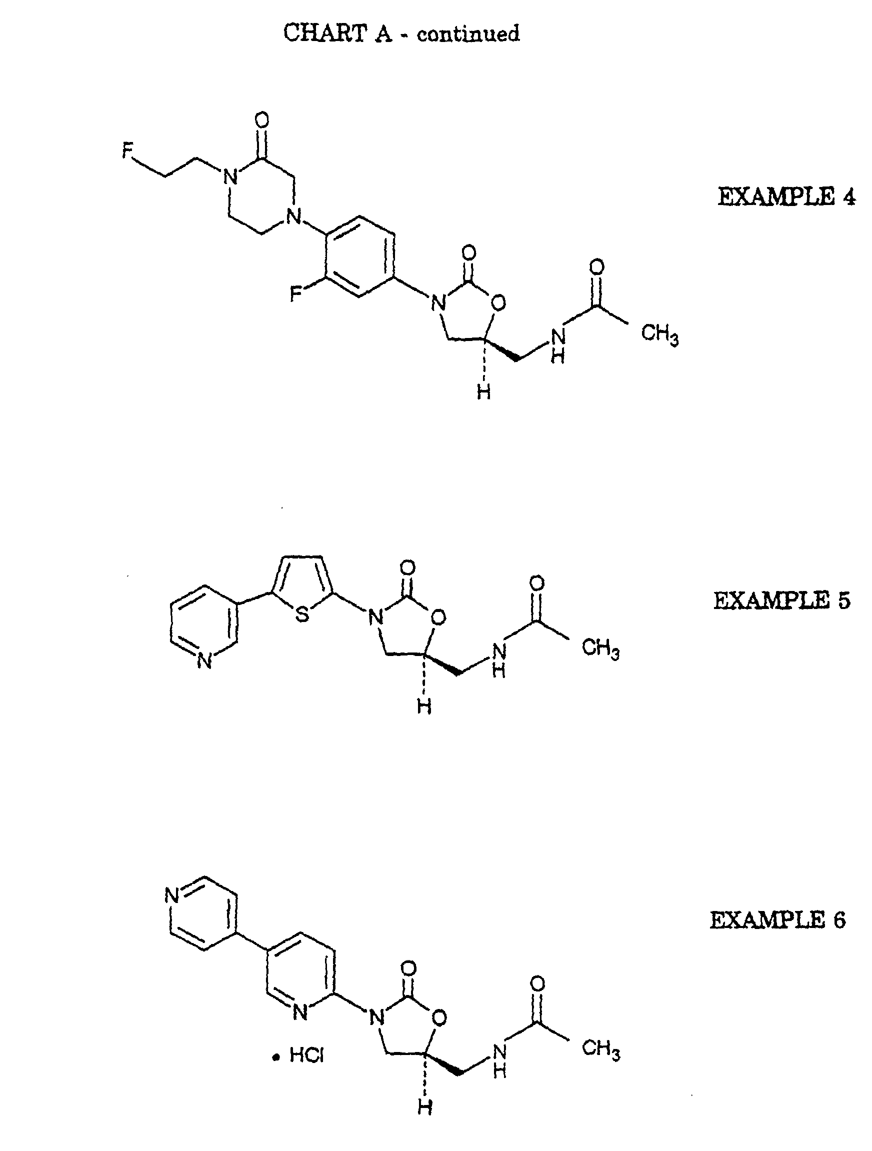

## Claims

1. Use of as oxazolidinone selected from:
(S)-N-[[3-[3-fluoro-4-[4-(hydroxyacetyl)-1-piperazinyl]-phenyl]-2-oxo-5-oxazolidinyl]methyl]acetamide,
(S)-N-[[3-[3-fluoro-4-(4-morpholinyl)phenyl]-2-oxo-5-oxazolidinyl]methyl]acetamide,
[4(S)-cis]-(-)-N-[[3-[3-fluoro-4-(tetrahydro-1-oxido-2H-thiopyran-4-yl)phenyl]-2-oxo-5-oxazolidinyl]methyl]acetamide,
N-((5*S*)-3-(3-fluoro-4-(4-(2-fluoroethyl)-3-oxopiperazin-1-yl)phenyl)-2-oxooxazolidin-5-ylmethyl)acetamide,
(S)-N-[[3-[5-(3-pyridyl)thiophen-2-yl]-2-oxo-5-oxazolidinyl]methyl]acetamide and
(*S*)-N-[[3-[5-(4-pyridyl)pyrid-2-yl]-2-oxo-5-oxazolidinyl]methyl]acetamide and pharmaceutically acceptable salts thereof for the manufacture of a medicament for use in treating arthritis.

2. Use according to claim 1 where the medicament is administered orally.

3. Use according to claim 2 where the daily dose of the oxazolidinone is from about 50 to about 1,200 mg/day.

4. Use according to claim 1 where the medicament is administered topically as a solution, cream, ointment, gel, lotion, suspension or emulsion.

5. Use according to claim 4 where the amount to be administered topically is from about 0.01% to about 10%.

6. Use according to claim 1 where the medicament is administered IV.

7. Use according to claim 6 where the amount to be administered IV is from about 50 to about 1,200 mg/day.

8. Use according to any preceding claim where the oxazolidinone is (S)-N-[[3-[3-fluoro-4-(4-morpholinyl)phenyl]-2-oxo-5-oxazolidinyl]methyl]acetamide.

## Patentansprüche

1. Verwendung eines Oxazolidinons, das ausgewählt ist aus
(S)-N-[(3-[3-Fluor-4-[4-(hydroxyacetyl)-1-piperazinyl]-phenyl]-2-oxo-5-oxazolidinyl]methyl]acetamid,
(S)-N-[[3-[3-Fluor-4-(4-morpholinyl)phenyl]-2-oxo-5-oxazolidinyl]methyl]acetamid,
[4(S)-cis]-(-)-N-[[3-[3-Fluor-4-(tetrahydro-1-oxido-2Hthiopyran-4-yl)phenyl]-2-oxo-5-oxazolidinyl]methyl]acetamid,
N-((5S)-3-(3-Fluor-4-(4-(2-fluorethyl)-3-oxopiperazin-1yl)phenyl)-2-oxooxazolidin-5-ylmethyl)acetamid,
(S)-N-[[3-[5-(3-Pyridyl)thiophen-2-yl]-2-oxo-5-oxazolidinyl]methyl]acetamid und
(S)-N-[[3-[5-(4-Pyridyl)pyrid-2-yl]-2-oxo-5-oxazolidinyl]methyl]acetamid, und pharmazeutisch akzeptabler Salze desselben zur Herstellung eines Medikaments zur Verwendung bei der Behandlung von Arthritis.

2. Verwendung nach Anspruch 1, wobei das Medikament oral verabreicht wird.

3. Verwendung nach Anspruch 2, wobei die Tagesdosis des Oxazolidinons etwa 50 bis etwa 1200 mg/Tag beträgt.

4. Verwendung nach Anspruch 1, wobei das Medikament topisch als Lösung, Creme, Salbe, Gel, Lotion, Suspension oder Emulsion verabreicht wird.

5. Verwendung nach Anspruch 4, wobei die topisch zu verabreichende Menge etwa 0,01 % bis etwa 10 % beträgt.

6. Verwendung nach Anspruch 1, wobei das Medikament iv verabreicht wird.

7. Verwendung nach Anspruch 6, wobei die iv zu verabreichende Menge etwa 50 bis etwa 1200 mg/Tag beträgt.

8. Verwendung nach einem der vorhergehenden Ansprüche, wobei das Oxazolidinon (S)-N-[[3-[3-Fluor-4-(4-morpholinyl)phenyl]-2-oxo-5-oxazolidinyl]methyl]acetamid ist.

## Revendications

1. Utilisation d'une oxazolidinone choisie entre :
(S)-N-[[3-[3-fluoro-4-[4-(hydroxyacétyl)-1-pipérazinyl]-phényl]-2-oxo-5-oxazolidinyl]-méthyl]-acétamide,
(S)-N-[[3-[3-fluoro-4-(4-morpholinyl)-phényl]-2-oxo-5-oxazolidinyl]-méthyl]-acétamide,
[4-(S)-cis]-(-)-N-[[3-[3-fluoro-4-(tétrahydro-1-oxydo-2H-thiopyranne-4-yl)-phényl]-2-oxo-5-oxazolidinyl]-méthyl]-acétamide,
N-((5S)-3-(3-fluoro-4-(4-(2-fluoréthyl)-3-oxopipérazine-1-yl)-phényl)-2-oxo-oxazolidine-5-ylméthyl)-acétamide,
(S)-N-[[3- [5-(3-pyridyl)-thiophène-2-yl]-2-oxo-5-oxazolidinyl]-méthyl]-acétamide, et
(S)-N-[[3-[5-(4-pyridyl)-pyrid-2-yl]-2-oxo-5-oxazolidinyl]-méthyl]-acétamide,
et leurs sels pharmaceutiquement acceptables pour la production d'un médicament destiné à être utilisé dans le traitement de l'arthrite.

2. Utilisation suivant la revendication 1, dans laquelle le médicament est administré par voie orale.

3. Utilisation suivant la revendication 2, dans laquelle la dose quotidienne de l'oxazolidinone est comprise dans l'intervalle d'environ 50 à environ 1200 mg/jour.

4. Utilisation suivant la revendication 1, dans laquelle le médicament est administré localement sous forme d'une solution, d'une crème, d'une pommade, d'un gel, d'une lotion, d'une suspension ou d'une émulsion.

5. Utilisation suivant la revendication 4, dans laquelle la quantité à administrer localement est comprise dans l'intervalle d'environ 0,01 % à environ 10 %.

6. Utilisation suivant la revendication 1, dans laquelle le médicament est administré par voie IV.

7. Utilisation suivant la revendication 6, dans laquelle la quantité à administrer par voie IV est comprise dans l'intervalle d'environ 50 à environ 1200 mg/jour.

8. Utilisation suivant l'une quelconque des revendications précédentes, dans laquelle l'oxazolidinone est le (S)-N-[[3-[3-fluoro-4-(4-morpholinyl)-phényl]-2-oxo-5-oxazolidinyl]-méthyl]-acétamide.
